# EUROPEAN PATENT APPLICATION

(11) **EP 4 664 471 A1**
(43) Date of publication of application: **17.12.2025**
(21) Application number: 24181300.5
(22) Date of filing: 11.06.2024
(51) Int. Cl.: G16H 30/20, G16H 30/40, G16H 50/20, G16H 40/63

(54) **CONTROLLING A USER INTERFACE FOR A MEDICAL IMAGING SYSTEM**

(71) Applicant: Koninklijke Philips N.V., 5656 AG Eindhoven (NL)
(72) Inventor: FRANZ, Astrid Ruth, Eindhoven (NL); LORENZ, Cristian, Eindhoven (NL); ORASANU, Eliza Teodora, 5656AG Eindhoven (NL); SCHMIDT-RICHBERG, Alexander, Eindhoven (NL)
(74) Representative: Philips Intellectual Property & Standards

(57) **Abstract**

A mechanism for identifying one or more target frames containing a representation of a target element. An indicator of the target element is received and used to identify the target frame(s) within stored medical video data

## Description

### FIELD OF THE INVENTION

The present invention relates to the field of medical imaging.

### BACKGROUND OF THE INVENTION

There is an increasing interest in and demand for medical imaging procedures and information derivable from medical imaging procedures. Image data produced from such medical imaging procedures is particularly useful for assessment and/or diagnosis of a (medical) subject.

A wide variety of medical imaging system are known, such as those that employ ultrasound imaging, magnetic resonance (MR) imaging, computed tomography (CT) imaging, position emission tomography (PET) imaging and so on.

One particularly common scenario in medical imaging is to compare image data, or findings from image data, to historic image data or findings. Typically, during a medical imaging procedure, relevant images or findings will be identified by a clinician. During any subsequent medical imaging procedure, a clinician may be able to obtain or retrieve these relevant images or findings.

There is an ongoing desire to improve the information made available to a clinician in performing a medical imaging procedure.

### SUMMARY OF THE INVENTION

The invention is defined by the claims.

According to examples in accordance with an aspect of the invention, there is provided a computer-implemented method of controlling a user interface for a medical imaging system. The computer-implemented method comprises: receiving an indicator of a target element for a target subject, wherein the target element is an annotation and/or an anatomical element; identifying, if present, one or more target frames of stored medical video data of the target subject, wherein each identified target frame contains a representation of the target element; and if at least one target frame is identified, controlling the user interface responsive to the identified one or more target frames.

The present disclosure provides a technique for identifying at least one target frame, of stored medical video data, that contains a representation of a target element. A user interface is then controlled responsive to the identified target frame(s), for instance, to provide a visual representation of the target frame and/or information derived therefrom.

The proposed approach advantageously facilitates provision of information from stored medical video data that may not otherwise have been immediately available to a clinician.

In particular, the proposed approach recognizes that, historically, only a small portion of previously acquired image data may have been available to an operator or clinician (e.g., only those parts of the image data that were explicitly saved or recorded by the clinician). This can prevent the possibility of tracking a later-identified abnormality or pathology that may have been present (e.g., but unrecognized) during earlier imaging procedures. By performing computer-implemented identification of the target frame(s) of stored medical video data responsive to an identified target element, then new target features can be identified in historic video data and made available to the operator/clinician for improved understanding of the historic condition of the medical subject. This provides valuable information for performing an assessment and/or diagnosis of the medical subject.

In some embodiments, the identifying, if present, one or more target frames comprises, responsive to the indicator of the target element, processing the stored medical video data to identify, if present, the one or more target frames.

In this way, the stored medical video data may be actively searched for the target element. This approach directly facilitates the identification of previously unnoticed or unrecorded target elements in the stored medical video data. This allows, for instance, for historic trends of target elements to be identified, monitored and used in the performance of an assessment and/or diagnostic task.

In some examples, the processing the stored medical video data comprises: defining first frames of the stored medical video data, wherein adjacent first frames are separated from one another by a predefined regular interval within the stored medical video data; processing each first frame to identify, if present, one or more second frames containing a representation of the target element; and if at least one second frame is identified, processing any identified second frame to identify the one or more target frames.

This approach achieves a faster and/or more efficient approach for processing the stored medical video data. In particular, it is recognized that, due to the nature of medical imaging, adjacent frames in stored medical video data will be extremely similar to one another. For instance, an anatomical element visible in one frame has a high likelihood of being visible in an adjacent frame. By effectively subsampling the stored medical video data using the proposed process, and only processing the subsampled frames (i.e., the first frames), more resource efficient identification of target frames can be achieved with little or negligible risk of missing a target element.

The predefined regular interval may represent a temporal interval, between capture of the adjacent first frames, of no less than 0.5 seconds, e.g., no less than 0.75 seconds. This approach recognizes that improved resource usage (e.g., power) can be achieved by temporally distancing the first frames from one another.

The predefined regular interval may represent a temporal interval, between capture of the adjacent first frames, of no more than 3 seconds, e.g., no more than 2 seconds. The present disclosure recognizes that there is unlikely to be significant change in the content of medical imaging data that are less than 3 seconds (e.g., less than 2 seconds) apart from one another. By setting the predefined regular interval to represent such a temporal interval, there is a significantly reduced risk of target elements, represented in the medical video data, being undetected or unidentified when processing only the first frames of the medical video data.

The predefined regular interval may represent a temporal interval, between capture of the adjacent first frames, of between 0.5 seconds and 3 seconds. This combines the advantages set out above.

In some examples, the step of processing each first frame comprises processing each first frame using a character or object detection algorithm to determine whether or not each first frame is a second frame. These techniques provide reliable and effective mechanisms for identifying whether or not there is a representation of the target element in each first frame, and therefore whether or not each first frame is a second frame.

In some examples, the step of processing any identified second frames comprises selecting one or more of the second frames as the one or more target frames.

The step of processing any identified second frames may comprise: processing the one or more second frames to identify whether or not the representation of the target element meets one or more predetermined criteria; and responsive to none of the one or more second frames meeting the one or more predetermined criteria: defining third frames of the stored medical video data, wherein each third frame is within a predetermined number of frames of at least one second frame; processing each third frame to identify, if present, one or more fourth frames containing a representation of the target element; and if at least one fourth frame is identified, selecting one or more of the fourth frames as the one or more target frames.

The identifying one or more frames may comprise: before receiving the indicator of the target element, processing the stored medical video data to identify, for each of a plurality of different candidate target elements, one or more frames, if present, containing a representation of the candidate target element; and responsive to the indicator of the target element: determining whether the indicator of the target element semantically matches one of the plurality of the candidate target elements; and responsive to determining that the indicator of the target element semantically matches one of the plurality of the candidate target elements, identifying, as the one or more target frames, the one or more frames containing the representation of the candidate target element semantically matching the target element.

This approach provides a technique that effectively precomputes/preprocesses and stores search results for a range of candidate target elements. By performing the precomputation, the step of identifying target frames can be made significantly more efficient, e.g., by avoiding the direct need to process the medical video data responsive to the indicated target element.

In some examples, the step of, if at least one target frame is identified, controlling a user interface responsive to the identified one or more target frames comprises: identifying, as a latest target frame, the target frame that was captured temporally latest amongst the one or more target frames; and controlling the user interface responsive to the identified latest target frame.

In some examples, the step of receiving an indicator of a target element comprises receiving a user input defining the indicator of the target element. This provides a user (e.g., operator or clinician) with control over the target element, thereby allowing the user to control the information made available to them by the user interface.

The step of receiving an indicator of a target element may comprise: controlling the user interface to provide a visual representation of a plurality of possible target elements; and receiving the user input indicating which of the plurality of possible target elements is to be the target element.

In some examples, the step of receiving an indicator of a target element comprises: receiving a medical image; and processing the medical image to identify, if present, one or more annotations and/or anatomical elements in the medical image; and if at least one annotation or anatomical element is identified in the medical image, producing an indicator of one of the one or more annotations as the indicator of the target element.

The medical image may be a medical image generated by the medical imaging system during an ongoing medical imaging session or procedure, e.g., a most recently generated medical image. This provides an individual with live and/or repeatedly updated information relevant to a current view produced by the medical imaging system.

The method may further comprise controlling the user interface to provide a visual representation of the medical image.

The target element may be a measurement of an anatomical structure and/or the anatomical element.

There is also proposed a computer program product comprising computer program code means which, when executed on a computing device having a processing system, cause the processing system to perform all of the steps of any herein disclosed method.

There is also proposed a processing system for a medical imaging system, the processing system being configured to: receive an indicator of a target element for a target subject, wherein the target element is an annotation and/or an anatomical element; identify, if present, one or more target frames of stored medical video data of the target subject, wherein each identified target frame contains a representation of the target element; and if at least one target frame is identified, control a user interface responsive to the identified one or more target frames.

The processing system may be appropriately adapted to carry out any herein proposed (computer-implemented) method and vice versa. The skilled person would be readily capable of modifying the processing system and/or computer-implemented method accordingly.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates a system in which embodiments can be employed;
Fig. 2 illustrates a proposed method;
Fig. 3 illustrates a variant to the proposed method;
Fig. 4 illustrates a step for use in the proposed method;
Fig. 5 illustrates another variant to the proposed method;
Fig. 6 illustrates a step for use in a proposed method; and
Fig. 7 illustrates yet another variant to the proposed method.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for identifying one or more target frames containing a representation of a target element. An indicator of the target element is received and used to identify the target frame(s) within stored medical video data.

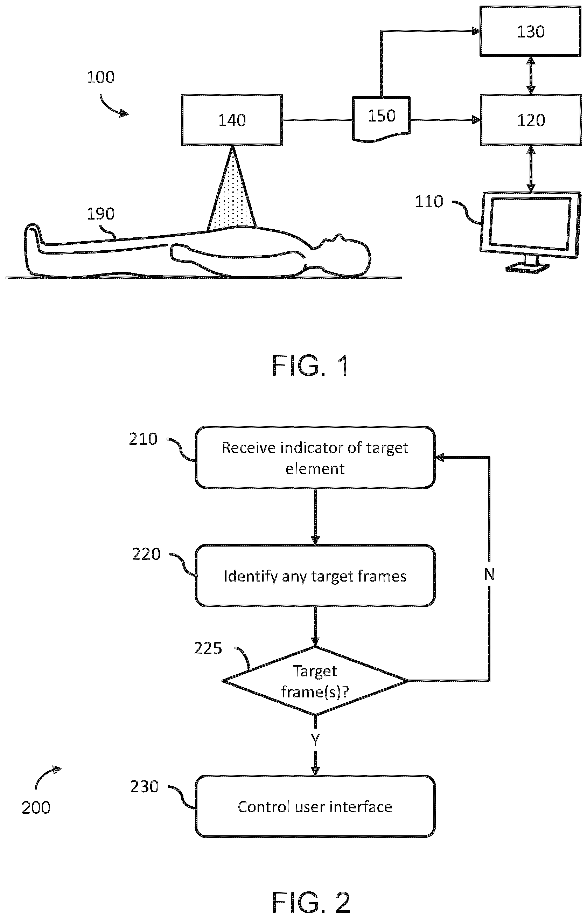

Fig. 1 illustrates a system 100 in which embodiments may be employed, for the purposes of improved contextual understanding. The system 100 comprises a user interface 110, a processing system 120; a memory 130 and a medical imaging system 140.

The user interface 110 is configured for interaction with an operator, such as a clinician for the medical imaging system. The operator is able to interact with the user interface 110 to control or adjust a user-perceptible output provided at the user interface. In particular, the user interface 110 may allow the operator to input data to the processing system and receive user-perceptible outputs that are controlled by the processing system.

In this way, the user interface may comprise one or more input elements (e.g., a mouse, keyboard, touchscreen and so on) and/or one or more output elements (e.g., a display, speaker system etc.). The function and operation of such elements are well known in the art.

The processing system 120 is configured to control the operation of the user interface. For instance, the processing system 120 may control the user-perceptiple output(s) provided by the user interface 110.

The memory 130 is configured to store data accessible to the processing system 120. For instance, the memory may store subject information (e.g., electronic medical records, historic imaging data and so on) that can be accessed by the processing system, e.g., responsive to a user input.

The medical imaging system 140 is configured to capture medical imaging data 150 (e.g., medical images) of a target subject 190. The medical imaging data captured by the medical imaging system 140 may be stored in the memory 130, e.g., for access by the processing system 120, and/or passed directly to the processing system 120 for further processing and/or display.

The medical imaging system 140 may be configured such that the complete medical imaging data produced during an imaging procedure is stored by default. In some examples, the configuration may require, after each imaging procedure, a confirmation from a user or operator whether or not the medical imaging data should be stored.

Suitable examples of medical imaging systems include: ultrasound systems; computed tomography (CT); magnetic resonance imaging (MRI) systems; X-ray systems; position emission tomography (PET) systems and so on. The operation and structure of such medical imaging systems are well known in the art.

In the context of the present disclosure, the medical imaging system 140 is configured to generate medical video data 150 and store the medical video data in the memory 130. As previously mentioned, the storage may be performed by default or only responsive to a confirmation or other action performed by an operator of the medical imaging system.

Medical video data is formed of a sequence or series of frames. Each frame is a different medical image, captured by a same medical imaging system, that was captured at/during or represents a different point or period of time. The order of the frames in the sequence indicates the relative position in time (i.e., temporal position) of each frame. More specifically, frames later in the sequence represents later points/periods of time than frames earlier in the sequence. Each frame can thereby be associated with a particular point/period of time.

Although only one medical imaging system 140 is illustrated in Fig. 1, it will be appreciated that, in practice, the memory 130 may receive medical video data from any number, e.g., a plurality, of different medical imaging systems. In particular, memory 130 may effectively be a databank storing a plurality of different instances of medical video data from different imaging sessions.

An operator of the user interface 110 may be able to request access to stored medical video data, e.g., of a particular imaging session and/or target subject. The processing system 120 may receive this request and, in response, retrieve the appropriate medical video data from the memory 130 and display the medical video data at the user interface 110. Techniques for performing such a procedure are well established in the art. This approach allows a clinician or operator of the user interface 110 to reference past imaging sessions for improved analysis and/or diagnosis of a target subject.

The user interface 110, processing system 120 and medical imaging system 140 may be further able to, during an imaging session of the medical imaging system 140, provide a live or near-live visual representation of medical imaging data captured by the medical imaging system during the imaging session. Thus, medical imaging data captured by the medical imaging system 140 during an imaging session may be visually represented or displayed at the user interface.

The present disclosure provides a procedure for identifying one or more target frames in (the) stored medical video data. This procedure can be carried out by the processing system. In particular, an indicator of a target element for a target subject is received. One or more frames of the medical video data that contain a representation of the target element are identified (if available/present). The user interface is controlled responsive to the/any identified frame(s), e.g., to provide information responsive to the/any identified frame(s) and/or display the/any identified frame(s).

In the context of the present disclosure, the target element is an element that may be represented in the medical video data, such as an annotation or an anatomical element in the medical video data.

In the context of the present disclosure, an anatomical element is any identifiable part of the anatomy or artificial anatomy that may be present within medical imaging/video data. The anatomical element may be a pathology (e.g., a tumor or growth), an artificial anatomical element (e.g., a stent or the like), a fetus, an anatomical organ or part (e.g., the heart, lungs, head, abdomen, stomach, spine etc.), a part of an anatomical organ (e.g., the mitral valve, lateral ventricle, cerebellum etc.) and so on.

In the context of the present disclosure, an annotation is an automated and/or clinician-defined element that does not directly represent the anatomy of the medical subject. For instance, the annotation may be a recorded value for a measurement of an anatomical element or a label for an anatomical element. The annotation may be textual (e.g., identifying a value or label) or non-textual (e.g., identifying a measuring element for measuring distance or an arrow pointing towards a particular element and so on). An annotation may, for instance, provide a value for a particular measurement. Example measurements include those used in fetal ultrasound or imaging techniques, such as a transverse cerebellar diameter (TCD), occipitofrontal diameter (OFD), head circumference (HC), and/or abdominal circumference (AC). A wide variety of other suitable measurements will be apparent to the skilled person.

The proposed techniques can, for instance, be used to support an ongoing imaging procedure with a medical subject. In particular, an indication of a target element can be produced (e.g., either by an operator of the imaging system or automatically) and used to control a display to the operator of the imaging system to support in the performance of an analysis and/or diagnosis performed using the ongoing imaging procedure.

Of course, it will be appreciated that the user interface may be further configured or controlled to provide a visual representative of imaging data for an ongoing medical imaging procedure at a same time as being controlled responsive to the identified frame(s).

Fig. 2 is a flowchart that illustrates a herein proposed generic computer-implemented method 200. The method 200 may be performed by the processing system.

The method 200 comprises a step 210 of receiving an indicator of a target element for a target subject. As previously indicated, the target element is an annotation and/or an anatomical element. A number of example approaches for receiving the indicator are provided later in this disclosure.

The method 200 further comprises a step 220 of identifying, if present, one or more target frames of stored medical video data of the target subject, wherein each identified target frame contains a representation of the target element. Step 220 may, for instance, comprise processing the stored medical video data responsive to the target element. More detailed and alternative approaches are described later.

If more than one instance of medical video data is available for the target subject, then a different iteration of step 220 may be repeated for each instance of medical video data. Alternatively, step 220 may be performed for all stored medical video data of the target subject in parallel.

Although a memory may store medical video data for a plurality of different subjects, the medical video data processed in step 220 is for a particular, specific target subject.

The method 200 further comprises a step 230 of, if at least one target frame is identified, controlling the user interface responsive to the identified one or more target frames.

Thus, step 230 is only performed if one or more target frames are identified. Accordingly, there may be a determination step 225 (between steps 220 and 230) that determines whether or not any target frames are identified in step 220. Responsive to a positive determination (i.e., at least one target frame is identified), the method moves to step 230. Otherwise, the method ends or, as illustrated, reverts back to step 210.

As a simple example, step 230 may comprise, if at least one target frame is identified, controlling the user interface to provide a visual representation of the identified target frame(s).

In a more complex example, step 230 may comprise, if at least one target frame is identified, processing the target frame(s) to extract or determine one or more pieces of information from the target frame(s) and controlling the user interface to provide a user-perceptiple output of the extracted piece(s) of information. Example pieces of information may include: a size of the target element; a shape of the target element; a certainty or confidence of the presence of the target element; a value or measurement represented by the target element (e.g., if the target element is an annotation); and so on.

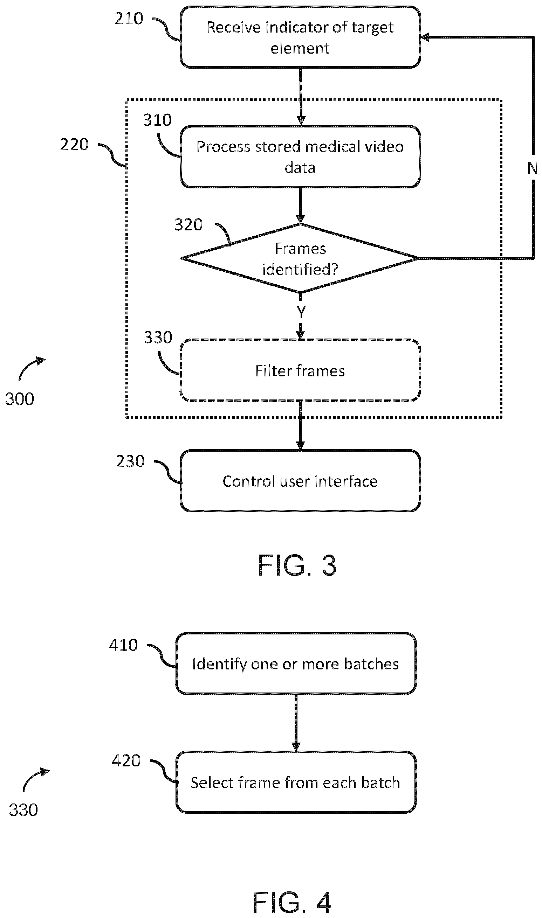

Fig. 3 illustrates one variant method 300 for the computer-implemented method 200 previously disclosed. In this approach, the method 300 comprises the steps 210, 220 and 230 previously disclosed.

The step 220 comprises a sub-step 310 of processing the stored medical video data to identify (if present) one or more target frames that contain a representation of the target element. Thus, the stored medical video data is actively processed responsive to the indicator of the target element to identify the target frame(s).

This approach allows for previously unidentified elements to be identified in the stored medical video data. This can allow, for instance, for anatomical elements that have not been previously identified, annotated or segmented from stored medical video data to be identified during subsequent investigation(s). Similarly, this can also allow for previous annotations, which have not been recorded or otherwise made searchable, in stored medical video data to be identified.

In some examples, sub-step 310 comprises using a character or object detection algorithm to process each frame of the stored medical data to determine whether or not each frame is a target frame. A character detection algorithm may be used, for instance, where the target element is a textual annotation. An object detection algorithm may be used where the target element is an anatomical element or non-textual annotation. It is common for such algorithms to indicate a measure of confidence that a frame contains a representation of a target element.

The step 220 may further comprise a sub-step 320 of determining whether or not one or more target frames are identified. This effectively integrates the previously described determination step 225 into step 220.

In some examples, if one or more target frames are identified (in step 310), then the step 220 moves to sub-step 330 of filtering the one or more target frames. The precise approach or technique applied for filtering the one or more target frames may be dependent upon the use-case scenario in which the proposed method 300 is employed, e.g., dependent upon the nature or type of the target element that is to be identified.

In other words, sub-step 330 comprises selecting and outputting only a subset of the target frames for future processing.

For instance, if the target element is an anatomical element, then sub-step 330 may comprise selecting only the identified target frame(s) that have the highest confidence of containing a representation of the target element or a confidence that exceeds a predetermined value.

As another example, if the target element is an anatomical element, then sub-step 330 may comprise selecting only the identified target frame(s) that have the largest representation of the target element or a target element that has a size that exceeds a predetermined size.

As another example, if the target element is an anatomical element, then sub-step 330 may comprise selecting only the highest quality identified target frame(s). Approaches for quantifying a quality of an image or frame are well known in the art, such as the approaches proposed and cited in Kong, Lingchao, et al. "Blind image quality prediction for object detection." 2019 IEEE Conference on Multimedia Information Processing and Retrieval (MIPR). IEEE, 2019.

However, it will be appreciated that sub-step 330 is optional, and may be skipped or bypassed. For instance, each frame identified in step 310 may instead be selected as a target frame.

Fig. 4 illustrates another example process for performing step 330.

In this approach, sub-step 330 comprises a step 410 of identifying one or more batches of target frames identified in step 310. A batch of target frames is an unbroken sub-sequence of target frames within the medical video data, i.e., a set of target frames that are temporally sequential to one another in the medical video data.

Sub-step 330 also comprises a step 420 of selecting and outputting a target frame from each batch to batch, i.e., filtering out all non-selected target frames.

The precise approach or technique applied for performing step 420 may be dependent upon the use-case scenario in which the proposed method 300 is employed, e.g., dependent upon the nature or type of the target element that is to be identified.

In one example, step 420 comprises selecting the temporally latest target frame from each batch of target frames. This approach is particularly advantageous when the target element is an annotation (such as a measurement) as it can be assumed that the temporally latest target frame that contains such an annotation represents the final (and therefore most accurate) determination of the value represented by the annotation.

In this way, the step of, if at least one target frame is identified, controlling a user interface responsive to the identified one or more target frames may comprise: identifying, as a latest target frame, the target frame that was captured temporally latest amongst the one or more target frames; and controlling the user interface responsive to the identified latest target frame.

In another example, step 420 comprises selecting one of the target frames that contains one or more predetermined GUI elements relevant for the target element. For instance, if the target element is an annotation of a (distance) measurement, then the one or more predetermined GUI elements may include two crosses representing calipers and a line between said crosses representing a distance tool. This approach can, for instance, facilitate automated identification of the frame(s) used when a distance measurement is performed.

In another example, step 420 comprises performing any selection approach previously disclosed for filtering the identified target frame(s), e.g., selecting responsive to: a measure of confidence that the target frame contains the representation of the target element a size of the representation of the target element; and/or an image quality of the target frame(s).

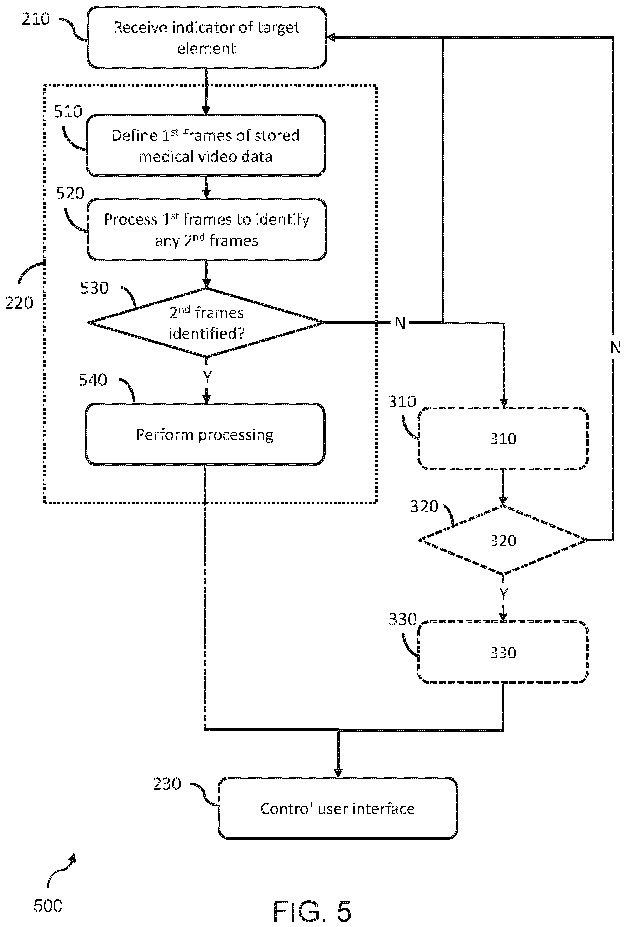

Fig. 5 illustrates a more complex variant method 500 for the computer-implemented method 200 previously disclosed. In this approach, the method 500 comprises the steps 210, 220 and 230 previously disclosed.

In this approach, step 220 comprises a sub-step 510 of defining first frames of the stored medical video data. Adjacent first frames are separated from one another by a predefined regular interval within the stored medical video data.

In this context, first frames are considered adjacent to one another when, if the first frames formed a first frame sequence that was ordered by time, they would be positioned immediately next to one another in the first frame sequence.

The predefined regular interval may represent a temporal interval, between capture of the adjacent first frames, of no less than 0.5 seconds, e.g., no less than 0.75 seconds. This approach recognizes that improved resource usage (e.g., power) can be achieved by temporally distancing the first frames from one another.

The predefined regular interval may represent a temporal interval, between capture of the adjacent first frames, of no more than 3 seconds, e.g., no more than 2 seconds. The present disclosure recognizes that there is unlikely to be significant change in the content of medical imaging data that are less than 3 seconds (e.g., less than 2 seconds) apart from one another. By setting the predefined regular interval to represent such a temporal interval, there is a significantly reduced risk of target elements, represented in the medical video data, being undetected or unidentified when processing only the first frames of the medical video data.

The predefined regular interval may represent a temporal interval, between capture of the adjacent first frames, of between 0.5 seconds and 3 seconds. This combines the advantages set out above.

In this way, the first frames may effectively be a gated version of the stored medical video data. Put another way, the first frames may effectively be a temporally down-sampled version of the medical video data, e.g., effectively reducing the number of frames-per-second represented by the first frames compared to the stored medical video data.

The step 220 here further comprises a sub-step 520 of processing each first frame to identify, if present, one or more second frames containing a representation of the target element. Thus, any first frame that contains a representation of the target element is identified as a second frame. Appropriate approaches for processing a frame to identify whether or not a representation of a target element is present are described elsewhere in this disclosure, but may generally include using a character or object detection algorithm to determine whether or not each first frame is a second frame.

The step 220 further comprises a step 540, if at least one second frame is identified, processing any identified second frame to identify the one or more target frames.

It will therefore be appreciated that step 220 may effectively comprise a determination step 530 of determining whether or not any second frames are identified. Responsive to at least one second frame being identified, then step 220 may perform step 540 of processing the identified second frame(s) to identify the one or more target frames.

Response to no second frame being identified, the method 500 may revert back to step 210. Alternatively, the method may perform steps 310, 320 and 330 previously described, e.g., to process every frame in the stored medical video data (rather than only the first frames). This latter approach reduces a risk that target frames will be overlooked or missed, whilst still benefiting from the advantage of reduced processing resource. Both alternatives are illustrated in Fig. 3.

In a simple example, and if performed, the step 540 of processing any identified second frames may comprise identifying all second frames as the one or more target frames.

In other examples, step 540 comprises performing further processing the second frames to identify the target frame(s).

By way of example, step 540 may comprise selecting one or more of the second frames as the one or more target frames. For instance, sub-step 540 may comprise filtering the second frame(s), i.e., selecting and outputting only a subset of the second frames as the target frame(s) for future processing. Suitable approaches for filtering frames have previously been described in the context of performing sub-step 330, and may be employed to similar effect to perform sub-step 540.

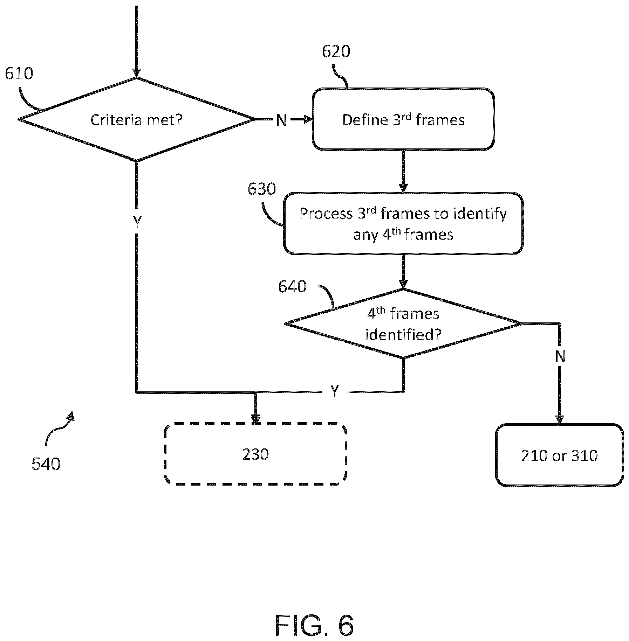

Fig. 6 illustrates an alternative approach to performing sub-step 540 on the second frame(s).

In this approach sub-step 540 comprises a sub-step 610 of processing the one or more second frames to identify whether or not the representation of the target element meets one or more predetermined criteria.

The one or more predetermined criteria may each be a criterion that the representation of the target element has a desired characteristic. It will be appreciated that the desired characteristic may vary depending upon the use-case scenario.

By way of example, in a scenario in which the target element is an annotation of a measurement performed by a clinician, it is common for the color of the annotation to change (in the medical video data) when a final decision or determination of the measurement has been made by the clinician. In this scenario, a suitable predetermined criterion would be that the color of the representation of the target element is a predetermined color (i.e., matching a color that indicates a final decision or determination of the measurement has been made).

As another example, in a scenario in which the target element is an anatomical element, then the predetermined criteria may be that the size of the representation is no less than a predetermined minimum size. This ensures that the target element is appropriately visible in the second frame(s).

Responsive to a positive determination in step 610, i.e., at least one second frame meets the one or more predetermined criteria, then step 540 may end (e.g., and move to step 230).

Responsive to a negative determination in step 610, i.e., none of the one or more second frames meets the one or more predetermined criteria, then the method may move to step 620.

Step 620 comprises defining third frames of the stored medical video data. Each third frame is within a predetermined number of frames of at least one second frame (within the stored medical video data). By way of example, each third frame may be a frame that lies within a number of frames that represents 1 second with respect to the second frame.

After performing step 620, the method moves to step 630 of processing each third frame to identify, if present, one or more fourth frames containing a representation of the target element. Thus, if present, one or more of the third frame(s) is/are selected as a fourth frame if they contain a representation of the target element. Step 630 may be performed in a similar manner to previously described approaches for any frames that contain a representation of the target element, e.g., using a character or object detection algorithm to determine whether or not each third frame is a fourth frame.

If at least one fourth frame is identified, e.g., if identified in a step 640, then the method may comprise selecting one or more of the fourth frames as the one or more target frames. This may comprise filtering the fourth frame(s), i.e., selecting and outputting only a subset of the fourth frames as the target frame(s) for future processing. Suitable approaches for filtering frames have previously been described in the context of performing sub-step 330, and may be employed to similar effect in this approach.

If no fourth frame is identified, the method may revert to step 210 and/or move to step 310 (if performed).

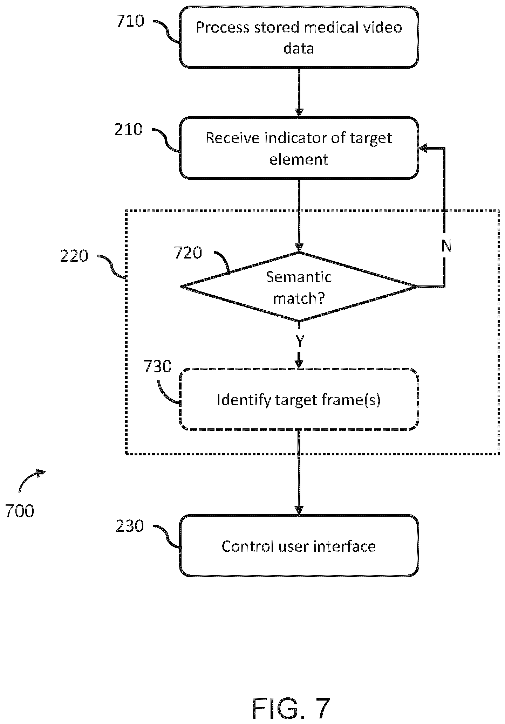

Fig. 7 illustrates another variant method 700 for the computer-implemented method 200 previously disclosed. In this approach, the method 500 comprises the steps 210, 220 and 230 previously disclosed.

Method 700 effectively relies upon a recognition that it is possible to precompute and store search results for a wide variety (e.g., all) available measurements and structures in the stored medical video data.

Accordingly, method 700 comprises a step 710 of before receiving the indicator of the target element, processing the stored medical video data to identify, for each of a plurality of different candidate target elements, one or more frames, if present, containing a representation of the candidate target element.

Each candidate target element represents a possible or desirable target element that may be present in the stored medical video data. More particularly, each candidate target element is an annotation and/or anatomical element that may be represented within the stored medical video data.

Approaches for performing step 710 to identify any frames containing a representation of the candidate target element may make use of an appropriately configured character or object detection algorithm to determine whether or not each frame of the stored medical video data comprises the candidate target element. Any appropriately identified frames may be labelled as containing the candidate target element.

The method 700 further comprises performing step 720 responsive to the indicator of the target element. Step 720 comprises determining whether the indicator of the target element semantically matches one of the plurality of the candidate target elements. Approaches for determining a semantic match between two target elements are established in the art.

For instance, each target element or frame may be associated with a classification or label, and a semantic matching technique may be used to determine whether or not the corresponding classifications/labels match, e.g., using techniques set out by Criunchiglia, Fausto, Mikalai Yatskevich, and Pavel Shvaiko. "Semantic matching: Algorithms and implementation." Journal on data semantics IX. Springer Berlin Heidelberg, 2007 or similar.

The method further comprises a step 730 of, responsive to determining that the indicator of the target element semantically matches one of the plurality of the candidate target elements, identifying, as the one or more target frames, the one or more frames containing the representation of the candidate target element semantically matching the target element.

Of course, in some examples, the identified one or more frames may undergo a filtering process for selecting one or more of the second frames as the one or more target frames. For instance, this process may comprise filtering the frame(s), i.e., selecting and outputting only a subset of the frames as the target frame(s) for future processing. Suitable approaches for filtering frames have previously been described in the context of performing sub-step 330, and may be employed to similar effect in this embodiment.

Previously described embodiments make use of an indicator of a target element. Example techniques for receiving or otherwise obtaining the indicator of the target element are hereafter described, together with some example use-case scenarios.

In some examples, the step of receiving an indicator of a target element comprises receiving a user input (e.g., at the user interface) defining the indicator of the target element. In this way, an operator or user is able to control or define the target element.

In at least one example, the step of receiving an indicator of a target element comprises: controlling the user interface to provide a visual representation of a plurality of possible target elements; and receiving the user input indicating which of the plurality of possible target elements is to be the target element. In this way, the indicator may be a selection of a predetermined list or set of possible target element.

The provision of the visual representation of the plurality of possible target elements may, for instance, be performed responsive to a second user input received at the user interface, e.g., a user selecting a button at the user interface. The visual representation of the plurality of possible target elements may take the form of one or more drop down menus.

In some examples, different sets of the plurality of possible target elements may be visually represented responsive to the receipt of different second user inputs (e.g., different buttons being selected). For instance, one second user input may trigger the provision of a visual representation of a first set of possible target elements, where a different second user input may trigger the provision of a visual representation of a second (different) set of possible target elements. The first set may, for instance, comprise only annotations. The second set may, for instance, comprise only anatomical elements.

In at least one example, the step of receiving an indicator of a target element comprises receiving a computer-generated indicator. For instance, in some examples, the processing system may be configured to process medical image data (e.g., a most recently available instance of medical image data produced by the medical imaging system) to identify a target element in the medical image data. This may be performed, for instance, using a segmentation, classification, character detection or object detection algorithm.

As a working example, the step of receiving an indicator of the target element may comprise receiving a medical image; processing the medical image to identify, if present, one or more annotations and/or anatomical elements in the medical image; and if at least one annotation or anatomical element is identified in the medical image, producing an indicator of one of the one or more annotations as the indicator of the target element.

A number of herein proposed embodiments make use of a character or object detection algorithm to process a frame of medical video data (e.g., a medical image). In particular, such approaches are used to determine whether or not a frame contains a representation of a target element.

A wide variety of suitable character or object detection algorithms are well known in the art. These include object detection algorithms such as that proposed by Redmon, Joseph, et al. "You only look once: Unified, real-time object detection." Proceedings of the IEEE conference on computer vision and pattern recognition. 2016; or those mentioned by Kaur, Amrita, et al. "A survey on deep learning approaches to medical images and a systematic look up into real-time object detection." Archives of Computational Methods in Engineering (2021): 1-41.

Character detection algorithms, also known as optical character recognition algorithms and/or text detection algorithms, are well known in the art. Examples include those indicated by Ye, Qixiang, and David Doermann. "Text detection and recognition in imagery: A survey." IEEE transactions on pattern analysis and machine intelligence 37.7 (2014): 1480-1500 or A. P. Tafti, et al.: OCR as a Service: An Experimental Evaluation of Google Docs OCR, Tesseract, ABBYY FineReader, and Transym. In: G. Bebis et al. (Eds.): ISCV 2016, Part I, LNCS 10072, pp. 735-746, 2016.

Some examples of character detection algorithms or object detection algorithms are or employ machine-learning algorithms. A machine-learning algorithm is any self-training algorithm that processes input data in order to produce or predict output data. Here, the input data comprises a frame of medical video data (e.g., a medical image) and the output data comprises an indicator of whether or not the frame of medical video data contains a representation of the target element.

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian models are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

A decision tree algorithm processes input data through a tree of nodes. In the tree of nodes, each successive node splits into two or more further nodes until reaching a terminal or end node. When performing the decision tree algorithm using the tree of nodes, at each node, a decision is made as to which further node to move to next based on the input data. The end node defines the outcome of the decision tree algorithm, and therefore the machine-learning algorithm.

Methods of training a machine-learning algorithm are well known. Typically, such methods comprise obtaining a training dataset, comprising training input data entries and corresponding training output data entries.

For some machine-learning algorithms, such as a neural network, training is performed by applying an initialized machine-learning algorithm to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

Other approaches for training machine-learning algorithms (e.g., decision trees) are known in the art. For instance, decision trees are often trained using a decision tree builder or learning techniques, such as those set out by Suthaharan, Shan, and Shan Suthaharan. "Decision tree learning." Machine Learning Models and Algorithms for Big Data Classification: Thinking with Examples for Effective Learning (2016): 237-269 or Ruggieri, Salvatore. "Yadt: Yet another decision tree builder." 16th IEEE International Conference on Tools with Artificial Intelligence. IEEE, 2004.

The training input data entries correspond to example frames of medical video data (e.g., example medical images). The training output data entries correspond to an indicator of whether or not the frame of medical video data contains a representation of the target element.

The skilled person would be readily capable of developing a processing system for carrying out any herein described method. Thus, each step of the flow chart may represent a different action performed by a processing system, and may be performed by a respective module of the processing system.

Embodiments may therefore make use of a processing system. The processing system can be implemented in numerous ways, with software and/or hardware, to perform the various functions required. A processor is one example of a processing system which employs one or more microprocessors that may be programmed using software (e.g., microcode) to perform the required functions. A processing system may however be implemented with or without employing a processor, and also may be implemented as a combination of dedicated hardware to perform some functions and a processor (e.g., one or more programmed microprocessors and associated circuitry) to perform other functions.

Examples of processing system components that may be employed in various embodiments of the present disclosure include, but are not limited to, conventional microprocessors, application specific integrated circuits (ASICs), and field-programmable gate arrays (FPGAs).

In various implementations, a processor or processing system may be associated with one or more storage media such as volatile and non-volatile computer memory such as RAM, PROM, EPROM, and EEPROM. The storage media may be encoded with one or more programs that, when executed on one or more processors and/or processing systems, perform the required functions. Various storage media may be fixed within a processor or processing system or may be transportable, such that the one or more programs stored thereon can be loaded into a processor or processing system.

It will be understood that disclosed methods are preferably computer-implemented methods. As such, there is also proposed the concept of a computer program comprising code means for implementing any described method when said program is run on a processing system, such as a computer. Thus, different portions, lines or blocks of code of a computer program according to an embodiment may be executed by a processing system or computer to perform any herein described method.

There is also proposed a non-transitory storage medium that stores or carries a computer program or computer code that, when executed by a processing system, causes the processing system to carry out any herein described method.

In some alternative implementations, the functions noted in the block diagram(s) or flow chart(s) may occur out of the order noted in the figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. The mere fact that certain measures are recited in mutually different dependent claims does not indicate that a combination of these measures cannot be used to advantage.

In the claims, the word "comprising" does not exclude other elements or steps, and the indefinite article "a" or "an" does not exclude a plurality. If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

A single processor or other unit may fulfill the functions of several items recited in the claims. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. A computer-implemented method (200, 300, 500, 700) of controlling a user interface (110) for a medical imaging system, the computer-implemented method comprising:
receiving (210) an indicator of a target element for a target subject, wherein the target element is an annotation and/or an anatomical element;
identifying (220), if present, one or more target frames of stored medical video data of the target subject, wherein each identified target frame contains a representation of the target element; and
if at least one target frame is identified, controlling (230) the user interface responsive to the identified one or more target frames.

2. The computer-implemented method of claim 1, wherein the identifying, if present, one or more target frames comprises, responsive to the indicator of the target element, processing (310) the stored medical video data to identify, if present, the one or more target frames.

3. The computer-implemented method of claim 2, wherein the processing the stored medical video data comprises:
defining (510) first frames of the stored medical video data, wherein adjacent first frames are separated from one another by a predefined regular interval within the stored medical video data;
processing (520) each first frame to identify, if present, one or more second frames containing a representation of the target element; and
if at least one second frame is identified, processing (540) any identified second frame to identify the one or more target frames.

4. The computer-implemented method of claim 3, wherein the predefined regular interval represents a temporal interval, between capture of the adjacent first frames, of between 0.5 seconds and 3 seconds.

5. The computer-implemented method of claim 3 or 4, wherein the step of processing (510) each first frame comprises processing each first frame using a character or object detection algorithm to determine whether or not each first frame is a second frame.

6. The computer-implemented method of any one of claims 3 to 5, wherein the step of processing (540) any identified second frames comprises selecting one or more of the second frames as the one or more target frames.

7. The computer-implemented method of any one of claims 3 to 5, wherein the step of processing (540) any identified second frames comprises:
processing (610) the one or more second frames to identify whether or not the representation of the target element meets one or more predetermined criteria; and
responsive to none of the one or more second frames meeting the one or more predetermined criteria:
defining (620) third frames of the stored medical video data, wherein each third frame is within a predetermined number of frames of at least one second frame;
processing (630) each third frame to identify, if present, one or more fourth frames containing a representation of the target element; and
if at least one fourth frame is identified, selecting (640) one or more of the fourth frames as the one or more target frames.

8. The computer-implemented method (700) of claim 1, wherein the identifying one or more frames comprises:
before receiving the indicator of the target element, processing (710) the stored medical video data to identify, for each of a plurality of different candidate target elements, one or more frames, if present, containing a representation of the candidate target element; and
responsive to the indicator of the target element:
determining (720) whether the indicator of the target element semantically matches one of the plurality of the candidate target elements; and
responsive to determining that the indicator of the target element semantically matches one of the plurality of the candidate target elements, identifying (730), as the one or more target frames, the one or more frames containing the representation of the candidate target element semantically matching the target element.

9. The computer-implemented method of any one of claims 1 to 8, wherein the step of, if at least one target frame is identified, controlling (230) a user interface responsive to the identified one or more target frames comprises:
identifying, as a latest target frame, the target frame that was captured temporally latest amongst the one or more target frames; and
controlling the user interface responsive to the identified latest target frame.

10. The computer-implemented method of any one of claims 1 to 9, wherein the step of receiving an indicator of a target element comprises receiving a user input defining the indicator of the target element.

11. The computer-implemented method of claim 10, wherein the step of receiving an indicator of a target element comprises:
controlling the user interface to provide a visual representation of a plurality of possible target elements; and
receiving the user input indicating which of the plurality of possible target elements is to be the target element.

12. The computer-implemented method of any one of claims 1 to 11, wherein the step of receiving an indicator of a target element comprises:
receiving a medical image; and
processing the medical image to identify, if present, one or more annotations and/or anatomical elements in the medical image; and
if at least one annotation or anatomical element is identified in the medical image, producing an indicator of one of the one or more annotations as the indicator of the target element.

13. The computer-implemented method of any one of claims 1 to 12, wherein the target element is a measurement of an anatomical structure and/or the anatomical element.

14. A computer program product comprising computer program code means which, when executed on a computing device having a processing system, cause the processing system to perform all of the steps of the method according to any one of claims 1 to 13.

15. A processing system (120) for a medical imaging system (100), the processing system being configured to:
receive (210) an indicator of a target element for a target subject, wherein the target element is an annotation and/or an anatomical element;
identify (220), if present, one or more target frames of stored medical video data of the target subject, wherein each identified target frame contains a representation of the target element; and
if at least one target frame is identified, control (230) a user interface (110) responsive to the identified one or more target frames.
